(19)

Europäisches
Patentamt
European
Patent Office
Office européen
des brevets

(11) **EP 4 585 923 A1**

(12) **EUROPEAN PATENT APPLICATION**
published in accordance with Art. 153(4) EPC

(43) Date of publication:
    **16.07.2025  Bulletin 2025/29**

(51) International Patent Classification (IPC):
    **G01N 33/53** (2006.01)

(21) Application number: **23862340.9**

(22) Date of filing: **04.09.2023**

(52) Cooperative Patent Classification (CPC):
    **C12Q 1/26; C12Q 1/32; G01N 21/64; G01N 33/493;
    G01N 33/53; G01N 33/564; G01N 33/58**

(86) International application number:
    **PCT/CN2023/116730**

(87) International publication number:
    **WO 2024/051646 (14.03.2024 Gazette 2024/11)**

(84) Designated Contracting States:
    **AL AT BE BG CH CY CZ DE DK EE ES FI FR GB
    GR HR HU IE IS IT LI LT LU LV MC ME MK MT NL
    NO PL PT RO RS SE SI SK SM TR**
    Designated Extension States:
    **BA**
    Designated Validation States:
    **KH MA MD TN**

(30) Priority: **06.09.2022  CN 202211084715**

(71) Applicant: **East China University of Science and
    Technology
    Shanghai 200237 (CN)**

(72) Inventors:
    • **ZHAO, Yuzheng
      Shanghai 200237 (CN)**
    • **YANG, Yi
      Shanghai 200237 (CN)**

    • **WANG, Congrong
      Shanghai 200237 (CN)**
    • **LI, Xie
      Shanghai 200237 (CN)**
    • **ZOU, Yejun
      Shanghai 200237 (CN)**
    • **ZHANG, Lijuan
      Shanghai 200237 (CN)**

(74) Representative: **Leonard, Thomas Charles et al
    Kilburn & Strode LLP
    Lacon London
    84 Theobalds Road
    London WC1X 8NL (GB)**

Remarks:
    The complete document including Reference
    Table(s) and the Sequence Listing(s) can be
    downloaded from the EPO website

(54) **METHOD AND KIT FOR DIAGNOSING MIDD**

(57)     The present invention relates to a method and a kit for diagnosing MIDD. Specifically, provided is a method for diagnosing MIDD, which comprises the following steps: (1) detecting lactic acid in urine; and (2) comparing same with the control level. The method of the present invention is simple and fast, has accurate results, and can be used for point-of-care detection of patients with MIDD.

Figure 6

## Description

This application claims priority to Chinese patent application No.202211084715.0, filed September 6, 2022, the entirety of which is hereby incorporated by reference herein.

TECHNICAL FIELD

The invention belongs to the field of biotechnology, and specifically relates to method and kit for diagnosing MIDD.

BACKGROUND

Diabetes is a chronic disease caused by abnormal glucose metabolism mechanism in human body, typically characterized by hyperglycemia. According to data from the International Diabetes Federation, as of 2019, there are approximately 463 million diabetic patients among people aged 20 to 79 years old. The total number of diabetic patients in China is approximately 116.4 million, ranking first in the world. Diabetes is currently incurable. Patients not only need to regularly monitor their blood glucose, but some even require lifelong insulin injections or hypoglycemic drugs. Therefore, diabetes has become one of the major diseases affecting the quality of life of Chinese residents. According to the cause of diabetes, it can be classified into T1DM, T2DM, gestational diabetes and special diabetes. T2DM is the main group of diabetic patients in my country, accounting for 53% of the total number of patients.

Diabetes is a disease caused by abnormality glucose metabolism regulatory mechanisms in human body, while lactate is the end product of glycolysis under anaerobic conditions. At present, a large number of literatures have reported that diabetic patients have abnormal lactate metabolism, but the relationship between the two remains unclear.

SUMMARY

Based on the shortcomings of the existing technology, the first aspect of the invention provides a reagent for detecting lactate in urine samples and its use in diagnosing MIDD.

The first aspect of the invention provides use of a reagent for detecting lactate in a urine-derived sample, and optionally a lactate standard, in the preparation of a kit for diagnosing MIDD (mitochondrial m.3243A>G mutation-associated diabetes) or for identifying MIDD in diabetic patients.

In one or more embodiments, the reagent for detecting lactate comprises the reagent required for detecting lactate by one or more methods selected from the group consisting of: chromatography, titration, colorimetry, enzymatic analysis, and optical probe method.

In one or more embodiments, the reagent for detecting lactate comprises the reagent for treating urine, such as 3NPH_HCI, EDC, and/or buffer. This treatment renders the lactate in the sample suitable for subsequent detection.

In one or more embodiments, the reagent for detecting lactate comprises the reagent that convert, enrich, isolate or identify lactate. Preferably, the reagent for detecting lactate comprises one or more selected from the group consisting of: an antibody, a lactate dehydrogenase, a lactate oxidase, a lactate binding protein or a functional variant thereof.

In one or more embodiments, the reagent for detecting lactate comprises one or more selected from the group consisting of: formic acid, acetonitrile, and isopropyl alcohol.

In one or more embodiments, the functional variant of the lactate binding protein comprises a lactate optical probe.

In one or more embodiments, the lactate optical probe is selected from the group consisting of: Laconic, Green Lindoblum, GEM-IL, eLACCO1.1, LiLac.

In one or more embodiments, the lactate optical probe comprises a fused lactate binding protein and an optically active polypeptide, wherein the optically active polypeptide is located in the sequence, at N-terminal or at C-terminal of the lactate binding protein.

In one or more embodiments, the lactate optical probe is as described in any embodiment of CN202011516287.5.

In one or more embodiments, lactate level is significantly increased in samples from MIDD patients compared to the control level. The control level is derived from lactate level in corresponding samples from non-MIDD patients or healthy subjects. In one or more embodiments, the control level is a value greater than or equal to $117 \pm 23\ \mu M$.

The second aspect of the invention further provides a kit for detecting lactate in urine, comprising a reagent for detecting lactate and a urine treatment reagent.

In one or more embodiments, the reagent for detecting lactate is as described in the first aspect herein.

In one or more embodiments, the urine treatment reagent comprises, but is not limited to: 3NPH_HCI, EDC, buffer.

In one or more embodiments, the buffer is a phosphate-based buffer or Tris, such as HEPES, PBS, etc.

In one or more embodiments, the kit further comprises a lactate standard.

In one or more embodiments, the reagent for detecting lactate comprises a reagent for converting, enriching, isolating or identifying lactate. Preferably, the reagent comprises one or more selected from the group consisting of: antibodies, lactate dehydrogenase, lactate oxidase, lactate binding proteins or functional variants thereof, formic acid, acetonitrile, isopropyl alcohol.

The invention further provides a kit for detecting lactate in urine, comprising the lactate optical probe and buffer described in any embodiment of the first aspect of the invention.

In one or more embodiments, the buffer is a phosphate-based buffer or Tris, such as HEPES, PBS, etc.

In one or more embodiments, the kit further comprises: a reagent for detecting mitochondrial m.3243A>G mutation, a reagent for detecting the blood glucose of the subject, and/or a reagent for detecting the urine glucose of the subject.

The invention also provides a method for diagnosing MIDD or identifying MIDD in diabetic patients, comprising: (1) detecting lactate in urine, and (2) comparing the same with the control level, wherein the lactate level in the urine of MIDD patients is higher than the control level.

In one or more embodiments, the step of detecting lactate is carried out by one or more methods selected from the group consisting of: chromatography, titration, colorimetry, enzymatic analysis, and optical probe method.

In one or more embodiments, the optical probe method comprises mixing urine with a lactate optical probe, detecting fluorescence intensity, and determining lactate content based on the fluorescence intensity.

In one or more embodiments, lactate level in urine from the MIDD patients is significantly increased compared with the control level. The control level is derived from lactate level in corresponding samples from non-MIDD patients or healthy subjects. Preferably, the control level is a value greater than or equal to $117 \pm 23$ μM.

In one or more embodiments, the lactate optical probe comprises a fused lactate binding protein and an optically active polypeptide, wherein the optically active polypeptide is located in the sequence, N-terminal or C-terminal of the lactate binding protein. Preferably, the lactate optical probe is as described in any embodiment of PCT/CN2020/137900.

Description of drawings

The invention will be further described below with reference to the accompanying drawings and examples.

Figure 1 shows the clinical characteristics of healthy people, LADA, T2DM and MIDD in the invention;
Figure 2 is a flow chart of the probe-based point-of-care detection method for clinical samples;
Figure 3 shows the serum detection results of the FiLa-H probe; Figure 3(A) shows the serum lactate level, and Figure 3(B) shows the comparison of the detection results between UHPLC-MS and FiLa-H probe;
Figure 4 shows the serum detection results of the FiLa probe; Figure 4(A) shows the serum lactate level, and Figure 4(B) shows the comparison of the detection results between UHPLC-MS and FiLa-H probe;
Figure 5 shows the urine detection results of the FiLa-H probe; Figure 5(A) shows the urine lactate level, and Figure 5(B) shows the comparison of the detection results between UHPLC-MS and FiLa-H probe;
Figure 6 shows the comparison of lacate levels between three diseased samples and healthy samples;
Figure 7 is a comparison chart of lacate levels among three diseased samples.

DETAILED DESCRIPTION

When a value or range is given, the term "about" as used herein means that the value or range is within 20%, within 10% and within 5% of the given value or range.

The terms "comprise", "include", and equivalent forms thereof include the meaning of "contain" as well as "consist of", for example a composition "comprising" X may consist of X alone or may contain other substances, such as X+Y.

Due to the lack of significant differentiation in the age of onset and clinical manifestations of patients, patients with MIDD (a special type of diabetes caused by mitochondrial m.3243A>G mutation) are often misdiagnosed as T1DM or T2DM. By detecting the serum and urine samples of patients with Latent Autoimmune Diabetes in Adults (LADA, a subtype of T1DM), with T2DM (Type 2 Diabetes Mellitus), and with MIDD (MIDD, a special subtype of diabetes caused by the mitochondrial m.3243A>G mutation), the changes in lactate levels were analyzed. The invention found that increased urinary lactate is closely related to patients with MIDD. Therefore, lactate can be used as a potential screening marker for the disease to re-identify these clinical manifestations of diabetes (abnormally increased urinary lactate and blood glucose) and thus promote rapid screening of MIDD.

Therefore, the invention provides a method for diagnosing MIDD or identifying MIDD in diabetic patients, comprising: (1) detecting lactate in urine, and (2) comparing same with the control level, wherein the lactate level in the urine of MIDD patients is higher than the control level. Specifically, the lactate level in the urine of MIDD patients was significantly elevated compared with the control level.

In the invention, the control level is the reference urinary lactate level that can serve as a basis for diagnosis. Such level can be obtained by comparison between samples based on MIDD subjects and samples from healthy subjects or non-MIDD subjects. In addition, the control level may also be that of a healthy subject or a non-MIDD subject. The control level can be derived from a single subject or a group comprising at least two subjects. Reference level can be selected by those skilled in the art based on the desired sensitivity and specificity. In an exemplary embodiment, the control level is a urinary lactate level greater than or equal to $117 \pm 23 \mu M$.

In an exemplary embodiment, lactate is detected by chromatography and probe methods. However, those skilled in the art can understand that other methods for detecting lactate in the art can also be used in the invention, such as titration, colorimetry, enzymatic analysis, and optical probe methods. Other methods of detecting lactate are within the knowledge of those skilled in the art. Those skilled in the art are well known of the steps and reagents for these methods to detect lactate. Exemplary descriptions of the above methods may refer to: chromatography (such as HPLC), titration (Biomedical Chromatography 2012, 26(11): 1408-1415; Food Chemistry 2012, 135(3): 1078-1082), colorimetry, detection method based on hydrogen peroxide, enzyme cycling (Cell Stem Cell 2019, 25(6): 754-767; Annals of Epidemiology 2013, 23(12): 791-796; Analyst 1972, 97(151): 142- 145), enzyme-linked electrochemical analysis (Biochemistry Biophysics Reports 2016, 5: 35-54.), nuclear magnetic resonance technology NMR (Nature Chemical Biology 2016, 12(11): 937-943), liquid chromatography mass spectrometry LC-MS (The Journal of Clinical Investigation 2021, 131(2): e136055; Nature 2017, 551(7678): 115-118)

Detection of lactate

Colorimetry:

Lactate dehydrogenase (LDH) converts lactate into pyruvate, and oxidized coenzymes (such as NAD+) accept H+ and are reduced to reduced coenzymes (such as NADH). The absorbance of NADH is measured at a wavelength of 340 nm to calculate the lactate content in the sample. In the reaction system, the reaction catalyzed by LDH is reversible, so it is necessary to add hydrazine compounds and pyruvate to the buffer to form a stable complex so that the reaction proceeds in the right direction. Reagents required for the colorimetric method include but are not limited to: lactate dehydrogenase, NAD+, buffer (such as Tris), hydrazine hydrate, etc.

Detection method based on hydrogen peroxide:

Lactate oxidase is used to convert lactate into pyruvate and produce hydrogen peroxide, and then the level of hydrogen peroxide is measured to quantitatively detect lactate. For example, lactate analyzers based on amperometry and photometry, or converting hydrogen peroxide into other detectable substances (such as quinone imine, 3-aminophthalate, etc.) under the action of peroxidase. The reagents required include but are not limited to: lactate oxidase, peroxidase, buffer, etc.

Chromatography:

Lactate can be detected by a variety of chromatography, such as HPLC, LC-MS, UHPLC-MS, etc. Exemplarily, the steps of detecting lactate by UHPLC - MS include: mixing the sample or standard solution with 3NPH_HCl solution and EDC solution in sequence. The mixture is frozen and the supernatant is obtained by centrifugation for quantitative analysis. The supernatant is injected into the UHPLC system, separated with water, and eluted with a formic acid and acetonitrile/isopropanol gradient. The paired ions for lactate quantification are 224/137.

Optical probe method:

The invention relates to a method for the immediate detection of lactate in clinical samples based on a genetically encoded optical probe. The method comprises: contacting optionally diluted urine with a lactate optical probe, and quantifying lactate by detecting the fluorescence change of the lactate optical probe.

In the invention, lactate optical probe refers to a polypeptide probe that quantitatively detects lactate level through changes in optical properties. Such probe typically comprises a protein that identifies lactate (i.e., a lactate binding protein) and a protein that changes its optical properties in response to binding of the protein to lactate (i.e., an optically active polypeptide, such as a fluorescent protein). Typically, the lactate optical probe comprises one or more lactate binding proteins and one or more optically active polypeptides, wherein the one or more optically active polypeptides are located in the sequence, N-terminal or C-terminal of the one or more lactate binding proteins. A variety of optical probes for detection of lactate are well-known in the art, for example: PCT/CN2020/137900, Laconic (PLos one 2013, 8(2), e57712), Green Lindoblum (Scientific Reports 2020, 10, 19562), GEM - IL (Cell Reports Methods 2021, 1(7), 100092), eLACCO1.1(Nature Communications 2021, 12, 7058), LiLac (Nature Communications 2022, 13, 2919). Those skilled in the art can understand that, in addition to the optical probes used in the examples, other lactate optical probes can also be used in the invention for the detection of lactate level.

An exemplary lactate optical probe used in the invention is the optical probe described in PCT/CN2020/137900, the entirety of which is hereby incorporated by reference herein. The lactate optical probe comprises a lactate binding protein or a functional variant thereof and an optically active polypeptide or a functional variant thereof, wherein the optically active polypeptide or a functional variant thereof is located in the sequence, N-terminal or C-terminal of the one or more lactate binding proteins.

In one or more embodiments, the lactate binding protein has the sequence shown in SEQ ID NO: 1 or a functional fragment thereof, or has at least 70%, at least 80%, at least 85%, at least 90%, at least 95%, or at least 99% sequence identity with it and retains the lactate binding function. In a preferred embodiment, the lactate binding protein has amino acids 80-258 of the sequence as shown in SEQ ID No: 1 or has the sequence shown in SEQ ID No: 1, or a sequence that has at least 35%, 40%, 50%, 60%, 70%, 80%, 85%, 90%, 95%, 99% sequence identity with it and retains lactate binding function.

In one embodiment, the optically active polypeptide is a fluorescent protein or a functional fragment or a variant thereof that retains fluorescent responsiveness. In one embodiment, the fluorescent protein is selected from the group consisting of yellow fluorescent protein, green fluorescent protein, blue fluorescent protein, apple red fluorescent protein (such as cpYFP, cpGFP, cpBFP and cpmApple shown in SEQ ID NO: 2-5 of PCT/CN2020/137900), respectively as shown in SEQ ID NO: 4-7 in the sequence listing of this application.

In one embodiment, the lactate optical probe further comprises one or more linkers flanking the optically active polypeptide. The linker of the invention may be any amino acid sequence of any length. In one embodiment, the optically active polypeptide is flanked by a linker of no more than 5 amino acids, for example, a linker of 0, 1, 2, 3, 4 amino acids. In one embodiment, the linkers flanking the optically active polypeptide comprise amino acid Y. In one embodiment, linker Y is located at the N- terminal and/or C- terminal of the optically active polypeptide. In one embodiment, the lactate optical probe is shown as follows: the first part B1 of the lactate binding protein - Y - optically active polypeptide A - the second part B2 of the lactate binding protein. In one embodiment, the lactate optical probe does not comprise a linker.

In one embodiment, the optically active polypeptides are located at one or more sites of the lactate binding protein selected from the group consisting of: 93/94, 93/95, 93/96, 93/97, 94/95, 94/96, 94 /97, 95/96, 95/97, 96/97, 119/120, 119/121, 120/121, 137/138, 137/139, 137/140, 137/141, 138/139, 138/140 , 138/141, 139/140, 139/141, 140/141, 158/159, 158/160, 158/161, 159/160, 159/161, 160/161, 185/186, 185/187, 185 /188, 185/189, 185/190, 185/191, 186/187, 186/188, 186/189, 186/190, 186/191, 187/188, 187/189, 187/190, 187/191 , 188/189, 188/190, 188/191, 189/190, 189/191, 190/191, 208/209, 208/210, 209/210, 230/231, 230/232 and/or 231/232 , wherein the numbering corresponds to the full length of the lactate binding protein. Preferably, the optically active polypeptide is located at one or more sites of the lactate binding protein selected from the group consisting of: 185/186, 185/187, 185/188, 185/189, 185/190, 185/191, 186/187, 186/188, 186/189, 186/190, 186/191, 187/188, 187/189, 187/190, 187/191, 188/189, 188/190, 188/191, 189/190, 189/ 191 or 190/191. In one or more embodiments, the B1-A-B2 lactate optical probe of the invention may be a probe wherein cpYFP is located at 185/186, 185/187, 185/188, 185/189, 185/190, 185/191, 186/187, 186/188, 186/189, 186/190, 186/191, 187/188, 187/189, 187/190, 187/191, 188/189, 188/190, 188/191, 189/190, 189/191 or 190/191 of the lactate binding protein or a functional fragment thereof. In an exemplary embodiment, the B1-A-B2 lactate optical probe may be a probe wherein cpYFP is located at 185/186, 185/187, 185/188, 185/189, 185/190, 186/187, 186/188, 186/189, 186/190, 187/189, 189/191 and 190/191 of the lactate binding protein or a functional fragment thereof. In one or more embodiments, the functional fragment of the lactate binding protein is as shown in positions 80-258 of SEQ ID NO: 1.

The lactate binding protein in the lactate optical probe may have one or more mutations. The lactate optical probe comprising mutated lactate binding protein is applicable for the detection of lactate whether its response to lactate is higher or lower than that of an unmutated counterpart. Preferably, a lactate optical probe is applicable for detecting lactate when its response to lactate (refer to PCT/CN2020/137900) exceeds 1.2-fold of a control fluorescent protein or falls below 0.8-fold of the control fluorescent protein. In one embodiment, the mutation is located at position 185, 189 and/or 190 of the lactate binding protein or a functional fragment thereof. Exemplarily, in one or more embodiments, the mutations comprise: P189R and P190D, P189R and P190A, P189R and P1901, P189R and P190Q, P189R and P190N, P189D and P190D, P189D and P190E, P189D and P190V, P189D and P190L, P189D and P190F, P189D and P1901, P189D and P190Q, P189D and P190N, P189D and P190G, P189D and P190Y, P189D and P190W, P189E and P190R, P189E and P190A, P189E and P190V, P189E and P190Q, P189A and P190L, P189A and P190F, P189A and P190M, P189A, P189A and P190N, P189A and P190G, P189A and P190H, P189A and P190T, P189V and P190D, P189V and P190E, P189V and P190A, P189V, P189V and P190N, P189V and P190H, P189V and P190Y, P189L and P190V, P189L and P190F, P189L and P190M, P189L and P190G, P189L and P190H, P189F and P190D, P189F and P190L, P189F and P190F, P189F and P190I, P189F and P190N, P189F and P190H, P189F and P190Y, P189F and P190K, P189F and P190T, P189F and P190W, P1891 and P190R, P1891 and P190D, P1891 and P190A, P1891 and P190V, P1891 and P190M, P1891 and

P190Q, P189I and P190G, P189I and P190Y, P189I and P190S, P189I and P190T, P189M and P190R, P189M and P190D, P189M and P190E, P189M and P190F, P189M and P190G, P189M and P190S, P189M and P190W, P189C and P190D, P189C and P190E, P189C and P190F, P189C and P190I, P189C and P190M, P189C and P190C, P189C, P189C and P190H, P189C and P190Y, P189C and P190S, P189C and P190W, P190L, P190F, P190I, P190Q, P190N, P190K, P190T, P189Q and P190E, P189Q and P190A, P189Q and P190V, P189Q and P190M, P189Q and P190C, P189Q and P190Q, P189Q and P190H, P189Q and P190S, P189N and P190R, P189N and P190D, P189N and P190L, P189N and P190F, P189N and P190C, P189N, P189N and P190N, P189N and P190G, P189N and P190H, P189N and P190Y, P189N and P190T, P189G and P190V, P189G and P190F, P189G and P190M, P189G and P190C, P189G and P190G, P189G and P190H, P189G and P190K, P189G and P190W, P189H and P190R, P189H and P190D, P189H and P190E, P189H and P190L, P189H and P190S, P189Y and P190R, P189Y and P190L, P189Y and P190N, P189Y and P190H, P189Y and P190S, P189Y and P190T, P189K and P190D, P189K and P190E, P189K and P190V, P189K and P190L, P189K and P190F, P189K and P190I, P189K and P190M, P189K, P189K and P190Q, P189K and P190N, P189K and P190Y, P189K and P190K, P189K and P190T, P189S and P190E, P189S and P190A, P189S and P190L, P190F, P189S and P190M, P189S and P190C, P189S, P189S and P190Q, P189S and P190Y, P189S and P190K, P189S and P190S, P189T and P190R, P189T and P190D, P189T and P190M, P189T and P190C, P189T, P189T and P190Q, P189T and P190N, P189T and P190H, P189T and P190Y, P189T and P190K, P189T and P190W, P189W and P190A, P189W and P190V, P189W and P190F, P189W, P189W and P190Q, P189W and P190H, P189W and P190S, P189W and P190T, P189W and P190W. In some specific embodiments, the mutations further comprise M185F, M185Y, M185L, M185I, M185Q, M185G, M185H, M185A, M185P, M185N, M185C, M185W, M185S, M185V, M185D, M185T, M185R, M185E, or M185K; Preferably, the mutation further comprises M185F, M185Y, M185L, M185I, M185Q, M185G, M185H, M185A, M185N, M185C, M185W, M185S, M185V, M185D, M185T, M185R or M185K. In some specific embodiments, the mutations comprise P189R and P190A, P189D and P190D, P189D and P190E, P189D and P190Q, P189D and P190Y, P189A and P190N, P189A and P190G, P189V and P190H, P189F and P190I, P189F and P190N, P189F and P190K, P189I and P190D, P189I and P190A, P189I and P190V, P189I and P190M, P189M and P190R, P189M and P190E, P189M and P190F, P189M and P190G, P189M and P190S, P189C and P190E, P190Q, P189Q and P190M, P189Q and P190C, P189N and P190N, P189G and P190F, P189H and P190L, P189H and P190S, P189Y and P190L, P189K and P190V, P189K and P190T, P189S and P190A, P189S and P190M, P189S and P190Q, P189S and P190K, P189S and P190S, P189T and P190D, P189W and P190A, P189W and P190T, P189C and P190D, P189C and P190Y, P189N and P190Y, P189R and P190I, P189M and P190D, P189H and P190R, P189N, P189F and P190D, P189F and P190H, P189N and P190F, P189C and P190F, P189H and P190D, or P189S. In some specific embodiments, the mutations further comprise M185F, M185Y, M185L, M185I, M185Q, M185G, M185H, M185A, M185P, M185N, M185C, M185W, M185S, M185V, M185D, M185T, M185R, M185E, or M185K; Preferably, the mutation further comprises M185F, M185Y, M185L, M185I, M185Q, M185G, M185H, M185A, M185N, M185C, M185W, M185S, M185V, M185D, M185T, M185R or M185K.

In some embodiments, the mutations comprise P189S, P189C and P190D, P189C and P190Y, P189N and P190Y, P189R and P190I, P189M and P190D, P189H and P190R, P189N, P189F and P190D, P189F and P190H, P189N and P 190F, P189C and P190F, or P189H and P190D. In one or more embodiments, the mutations further comprise M185F, M185Y, M185L, M185I, M185Q, M185G, M185H, M185A, M185P, M185N, M185C, M185W, M185S, M185V, M185D, M185T, M185R, M185E, or M185K; Preferably, the mutation further comprises M185F, M185Y, M185L, M185I, M185Q, M185G, M185H, M185A, M185N, M185C, M185W, M185S, M185V, M185D, M185T, M185R or M185K.

In some embodiments, the mutations comprise: (1) P189C and P190D, P189M and P190D, P189F and P190D, or P189H and P190D, and optionally (2) M185F, M185Y, M185L, M185I, M185Q, M185G, M185H, M185A, M185P, M185N, M185C, M185W, M185S, M185V, M185D, M185T, M185R, M185E or M185K.

In some embodiments, the mutations comprise: (1) P189C and P190D, P189M and P190D, or P189H and P190D, and optionally (2) M185F, M185Y, M185L, M185I, M185Q, M185G, M185H, M185A, M185P, M185N, M185C, M185W, M185S, M185V, M185D, M185T, M185R, M185E or M185K; alternatively, the mutation comprises: (1) P189F and P190D, and optionally (2) M185F, M185Y, M185L, M185I, M185Q, M185G, M185H, M185A, M185N, M185C, M185W, M185S, M185V, M185D, M185T, M185R or M185K.

In an exemplary embodiment, the lactate optical probe may be a probe having cpYFP inserted at 185/189 of the lactate binding protein and one or more mutations selected from the group consisting of: P189R and P190D, P189R and P190A, P189R and P190I, P189R and P190Q, P189R and P190N, P189D and P190D, P189D and P190E, P189D and P190V, P189D and P190L, P189D and P190F, P189D and P190I, P189D and P190Q, P189D and P190N, P189D and P190G, P189D and P190Y, P189D and P190W, P189E and P190R, P189E and P190A, P189E and P190V, P189E and P190Q, P189A and P190L, P189A and P190F, P189A and P190M, P189A, P189A and P190N, P189A and P190G, P189A and P190H, P189A and P190T, P189V and P190D, P189V and P190E, P189V and P190A, P189V, P189V and P190N, P189V and P190H, P189V and P190Y, P189L and P190V, P189L and P190F, P189L and P190M, P189L and P190G, P189L and P190H, P189F and P190D, P189F and P190L, P189F and P190F, P189F and P190I, P189F and P190N, P189F and P190H, P189F and P190Y, P189F and P190K, P189F and P190T, P189F and P190W, P189I and P190R, P189I and P190D, P189I and P190A, P189I and P190V, P189I and P190M, P189I and P190Q, P189I and P190G,

P189I and P190Y, P1891 and P190S, P1891 and P190T, P189M and P190R, P189M and P190D, P189M and P190E, P189M and P190F, P189M and P190G, P189M and P190S, P189M and P190W, P189C and P190D, P189C and P190E, P189C and P190F, P189C and P190I, P189C and P190M, P189C and P190C, P189C, P189C and P190H, P189C and P190Y, P189C and P190S, P189C and P190W, P190L, P190F, P1901, P190Q, P190N, P190K, P190T, P189Q and P190E, P189Q and P190A, P189Q and P190V, P189Q and P190M, P189Q and P190C, P189Q and P190Q, P189Q and P190H, P189Q and P190S, P189N and P190R, P189N and P190D, P189N and P190L, P189N and P190F, P189N and P190C, P189N, P189N and P190N, P189N and P190G, P189N and P190H, P189N and P190Y, P189N and P190T, P189G and P190V, P189G and P190F, P189G and P190M, P189G and P190C, P189G and P190G, P189G and P190H, P189G and P190K, P189G and P190W, P189H and P190R, P189H and P190D, P189H and P190E, P189H and P190L, P189H and P190S, P189Y and P190R, P189Y and P190L, P189Y and P190N, P189Y and P190H, P189Y and P190S, P189Y and P190T, P189K and P190D, P189K and P190E, P189K and P190V, P189K and P190L, P189K and P190F, P189K and P190I, P189K and P190M, P189K, P189K and P190Q, P189K and P190N, P189K and P190Y, P189K and P190K, P189K and P190T, P189S and P190E, P189S and P190A, P189S and P190L, P189S and P190F, P189S and P190M, P189S and P190C, P189S, P189S and P190Q, P189S and P190Y, P189S and P190K, P189S and P190S, P189T and P190R, P189T and P190D, P189T and P190M, P189T and P190C, P189T, P189T and P190Q, P189T and P190N, P189T and P190H, P189T and P190Y, P189T and P190K, P189T and P190W, P189W and P190A, P189W and P190V, P189W and P190F, P189W, P189W and P190Q, P189W and P190H, P189W and P190S, P189W and P190T, P189W and P190W. In further embodiments, the mutations further comprise M185F, M185Y, M185L, M1851, M185Q, M185G, M185H, M185A, M185P, M185N, M185C, M185W, M185S, M185V, M185D, M185T, M185R, M185E, or M185K; Preferably, the mutation further comprises M185F, M185Y, M185L, M1851, M185Q, M185G, M185H, M185A, M185N, M185C, M185W, M185S, M185V, M185D, M185T, M185R or M185K.

In an exemplary embodiment, the lactate optical probe may be a probe having cpYFP inserted at 185/189 of the lactate binding protein and one or more mutations selected from the group consisting of: P189S, P189C and P190D, P189C and P190Y, P189N and P190Y, P189R and P190I, P189M and P190D, P189H and P190R, P189N, P189F and P190D, P189F and P190H, P189N and P190F, P189C and P190F, or P189H and P190D. In an exemplary embodiment, the lactate binding protein functional fragment is as shown in positions 80-258 of SEQ ID NO: 1, and the mutations are P189N, P189S, P189C and P190F, P189N and P190F, P189N and P190Y, P189H and P190R, P189R and P190I, P189F and P190H, P189C and P190Y, P189C and P190D, P189M and P190D, P189H and P190D, or P189F and P190D. In further embodiments, the mutations further comprise M185F, M185Y, M185L, M185I, M185Q, M185G, M185H, M185A, M185P, M185N, M185C, M185W, M185S, M185V, M185D, M185T, M185R, M185E, or M185K; Preferably, the mutation further comprises M185F, M185Y, M185L, M1851, M185Q, M185G, M185H, M185A, M185N, M185C, M185W, M185S, M185V, M185D, M185T, M185R or M185K.

In some specific embodiments, the lactate optical probe may be a probe having cpYFP inserted at 185/189 of the functional fragment of the lactate binding protein and having mutations, wherein the functional fragment of the lactate binding protein is as shown in positions 80-258 of SEQ ID NO: 1, and the mutations comprise: (1) P189C and P190D, P189M and P190D, P189F and P190D, or P189H and P190D, and optionally (2) M185F, M185Y, M185L, M1851, M185Q, M185G, M185H, M185A, M185P, M185N, M185C, M185W, M185S, M185V, M185D, M185T, M185R, M185E or M185K. Preferably, the mutations comprise: (1) P189C and P190D, P189M and P190D, or P189H and P190D, and optionally (2) M185F, M185Y, M185L, M185I, M185Q, M185G, M185H, M185A, M185P, M185N . M185G, M185H, M185A, M185N, M185C, M185W, M185S, M185V, M185D, M185T, M185R or M185K. Preferably, the lactate optical probe may be a probe having cpYFP inserted at 185/189 of the functional fragment of the lactate binding protein and having P189F and P190D mutations, or having cpYFP inserted at 185/189 of the functional fragment of the lactate binding protein and having M185L, P189H and P190D mutations.

In a specific embodiment, the lactate optical probe includes a lactate optical probe having the sequence shown in SEQ ID NO: 6-30, 34-40 in PCT/CN2020/137900. In an exemplary embodiment, the lactate optical probe comprises or consists of a variant of the amino acid sequence SEQ ID NO: 2 or 3 (SEQ ID NO: 30 or 35 in PCT/CN2020/137900). In one embodiment, the lactate optical probe comprises the sequence having at least 35%, at least 40%, at least 50%, at least 60%, at least 70%, at least 80%, at least 85%, at least 90%, at least 95%, or at least 99% sequence identity to the amino acid sequence of SEQ ID NO:2 or 3.

The lactate optical probe can be fused with other functional polypeptides. For example, the functional polypeptide is located at the N-terminal and/or C-terminal of the lactate optical probe. In some embodiments, the functional polypeptide comprises a tag for purification or a tag for immunoblotting. There can be linkers between optical probes and other functional polypeptides.

The terms "functional variant", "derivative", and "analog" used herein refer to protein that maintains essentially the same biological function or activity as the original polypeptide or protein (e.g., a lactate binding protein or a fluorescent protein). Functional variants, derivatives, or analogues of a polypeptide or protein (e.g., a lactate binding protein or a fluorescent protein) of the invention may be (i) a protein having one or more conservative or nonconservative amino acid residues (preferably conservative amino acid residues) substituted, whereas such substituted amino acid residues may or may not be encoded by the genetic code, or (ii) a protein having a substituted group in one or more amino acid residues, or (iii) a protein formed by the fusion of the mature protein to another compound, such as a compound that extends the half-life of the protein, such as polyethylene glycol, or (iv) a protein formed by the fusion of an additional amino acid sequence to this protein sequence (such as a secretory sequence or the sequence or protein used to purify this protein, or the fusion protein formed with an antigenic IgG fragment). According to the teaching herein, these functional variants, derivatives, and analogues belong to the common knowledge to those skilled in the art.

The difference of said analogues from the original polypeptide or protein may be a difference in amino acid sequence, a difference in modified form that does not affect the sequence, or both. These proteins include natural or induced genetic variants. Induced variants can be derived by various techniques, such as random mutagenesis by radiation or exposure to mutagens and can also be obtained by site directed mutagenesis or other known techniques in molecular biology.

In some embodiments, the process of detecting lactate includes the step of establishing a lactate standard curve. The determination of the lactate standard curve is a routine method in this field. It is a curve established by correspondingly detecting lactate standard of different known concentrations and correlating the concentrations with the detection results or calculation results derived from the detection results.

For lactate analysis based on optical probes, after contacting the optical probe with lactate in the sample, the appropriate excitation and emission wavelengths are selected according to the different fluorescent proteins to detect the fluorescence intensity, and quantitative analysis is performed based on the lactate standard curve.

In an exemplary embodiment, for fluorescent proteins with a single excitation wavelength (eg, cpBFP and cpmApple), the data processing procedure includes:

$$F = F_{Sample} - F_{BLK}$$

F represents the actual fluorescence intensity of a single channel, $F_{Sample}$ represents the fluorescence intensity of the sample expressing the probe, and $F_{BLK}$ represents the fluorescence intensity of the sample not expressing the probe. A standard curve was established using the fluorescence intensity of the standard, and then quantitative analysis of lactate in the sample was performed based on the standard curve. In an exemplary embodiment, for cpBFP, the excitation wavelength is 360nm BP 10nm and the emission wavelength is 450nm BP 10nm; for cpmApple, the excitation wavelength is 540nm BP 25nm and the emission wavelength is 590nm BP 20nm.

For multi-excitation wavelength fluorescent proteins (such as cpYFP and cpGFP), the data processing process includes:

$$F = F_{Sample} - F_{BLK}$$

$$R = F_{excitation\ wavelength\ 1} / F_{excitation\ wavelength\ 2}$$

F represents the actual fluorescence intensity of a single channel, $F_{Sample}$ represents the fluorescence intensity of the sample expressing the probe, and $F_{BLK}$ represents the fluorescence intensity of the sample not expressing the probe. $F_{excitation\ wavelength\ 1}$ represents the fluorescence intensity emitted by the probe at the emission wavelength (528 nm for cpYFP or cpGFP) after being excited at the first excitation wavelength (485nm BP 20nm for cpYFP). $F_{excitation\ wavelength\ 2}$ represents the fluorescence intensity emitted by the probe at the emission wavelength (528nm for cpYFP or cpGFP) after being excited at the second excitation wavelength (420nm BP 20nm for cpYFP). The first and second excitation wavelengths can be determined according to the spectral properties of the fluorescent protein carried by the probe.

R (Ratio) represents the fluorescence ratio of the probe. The bandwidth BP (band pass) of the filter represents the total range on both sides of the median, such as 485BP 20nm, which is 475-495nm.

$$[Lac] = Kd(R - R_{min}) / (R_{max} - R)$$

[Lac] represents the lactate level; $K_d$ represents the dissociation constant of the probe; $R_{min}$ and $R_{max}$ represent the fluorescence ratio of the probe protein without adding or adding saturated concentration of lactate respectively; R represents the fluorescence ratio of the sample.

Usually, before the detection of lactate, the sample can be pretreated to remove substances that may affect the detection. Such pretreatment methods can be tailored to the specific detection method. Those skilled in the art are aware of the procedures and required reagents for such pretreatment. For example, the method of using UHPLC-MS analysis is as described in Xie et al., 2021. Sample pretreatment includes: mixing the sample (such as blood, urine) with 3NPH_HCl solution and EDC solution in sequence, then freezing at -20°C and Take the supernatant for analysis. For another example, when using an optical probe to detect lactate in a sample, the sample (such as blood, urine) is first diluted with a buffer (such as HEPES), and then mixed with the optical probe to measure the fluorescence intensity.

In addition, the invention also provides a kit for detecting lactate in urine, which kit comprises a reagent for detecting lactate and an optional urine treatment reagent.

The terms lactate "detection substance", "detection reagent" and "reagent for detection of lactate" used herein are used interchangeably and all refer to substances that are specific to lactate and can be used to directly or indirectly detect the presence and/or content of lactate. In order to facilitate detection, the detection reagent of the invention can also be equipped with detectable labels. The detectable labels include but are not limited to: radioactive isotopes, fluorophores, chemiluminescent moieties, enzymes, enzyme substrates, enzyme cofactors, enzyme inhibitors, dyes, metal ions, ligands (such as biotin or half-antibodies), etc.

In some embodiments, the reagents for detecting lactate comprise reagents that convert, enrich, isolate, or identify lactate. As mentioned above, those skilled in the art can understand that methods for detecting lactate, including chromatography and probe methods, can be used in the invention, such as titration methods, colorimetric methods, enzymatic analysiss, and optical probe methods. Those skilled in the art are well known of the steps and reagents for these methods to detect lactate. Therefore, exemplarily, the reagents for detecting lactate herein are reagents used in the following methods: chromatography (such as HPLC), titration (Biomedical Chromatography 2012, 26(11): 1408-1415; Food Chemistry2012, 135(3):1078-1082), colorimetric method, detection method based on hydrogen peroxide, enzyme cycle method (Cell Stem Cell 2019, 25(6): 754-767; Annals of Epidemiology 2013, 23(12): 791-796; Analyst 1972, 97(151): 142-145), enzyme-linked electrochemical analysis method (Biochemistry Biophysics Reports 2016, 5: 35-54.), nuclear magnetic resonance technology NMR (Nature Chemical Biology 2016, 12(11) : 937-943), liquid phase mass spectrometry LC-MS (The Journal of Clinical Investigation 2021, 131(2): e136055; Nature 2017, 551(7678): 115-118). Reagents involved in the above methods include, but are not limited to: antibodies, lactate dehydrogenase, lactate oxidase, lactate binding protein or functional variants thereof. In an exemplary embodiment, the reagent for detecting lactate may also be one or more selected from the following: 3NPH_HCI, EDC, formic acid, acetonitrile, and isopropyl alcohol.

After using Urine treatment reagent, lactate in the sample may be more suitable for subsequent detection. Such pretreatment methods can be adjusted according to the specific detection method. Those skilled in the art are aware of the procedures and required reagents for such pretreatment. For example, the method of using UHPLC-MS analysis is as described in Xie et al., 2021. Sample pretreatment comprises: the sample (such as blood and urine) is sequentially mixed with the 3NPH_HCI solution and the EDC solution, then frozen at -20 °C and the supernatant is taken for analysis. For another example, when using an optical probe to detect lactate in a sample, the sample (such as blood, urine) is first diluted with a buffer (such as HEPES), and then mixed with the optical probe to measure the fluorescence intensity. In one or more embodiments, the urine treatment reagents include but are not limited to: 3NPH_HCI, EDC, buffer.

The kit may also comprise a buffer. The function of the buffer is to provide a stable buffer environment for the reactions involved in the detection of lactate. Those skilled in the art can select appropriate buffers based on experience, such as phosphate-based buffers (HEPES, PBS) or Tris, etc.

The kit may also comprise a reagent required for other auxiliary detection that may be involved in the diagnosis of MIDD, such as a reagent for detecting mitochondrial m.3243A>G mutation (such as PCR primers), a reagent for detecting the subject's blood glucose (such as glucose oxidase), a reagent for detecting the subject's urine glucose (such as glucose oxidase). These are within the scope of knowledge of those skilled in the art.

The invention also provides the use of the above-mentioned reagent for detecting lactate in preparing a kit for detecting MIDD or identifying MIDD in diabetic patients.

Concentrations, contents, percentages, and other values may be expressed with ranges available herein. It is also understood that use of these ranges is only for convenience and conciseness, which should be interpreted elastically to include values explicitly mentioned in the upper and lower limits of the range, but also to include all individual values or sub ranges included in the ranges.

The invention has the following beneficial effects:

The detection of body fluid samples based on probes does not require time-consuming sample preparation (i.e., pretreatment or purification), so it is very rapid and convenient. Generally, the entire process from sample pipetting to measurement takes about 1 minute for one sample and about 3 minutes for 96 samples in an automated microplate assay. These advantages make it a promising technology for metabolic diagnosis and screening.

Some embodiments of the invention:

1. Use of a reagent for detecting lactate in a urine-derived sample, or a lactate standard and a reagent for detecting lactate in a urine-derived sample, in the preparation of a kit for diagnosing **MIDD** or for identifying **MIDD** in diabetic patients.

2. The use according to item 1, wherein the reagent for detecting lactate comprises a reagent required for detecting lactate by one or more methods selected from the group consisting of: chromatography, titration, colorimetry, enzymatic analysis, optical probe method.

3. The use according to item 1 or 2, wherein the reagent for detecting lactate comprises a reagent for converting, enriching, separating or identifying lactate,
preferably, the reagent for detecting lactate comprises one or more reagents selected from the group consisting of: antibodies, lactate dehydrogenase, lactate oxidase, lactate binding proteins or functional variants thereof, formic acid, acetonitrile, isopropyl alcohol.

4. The use according to item 3, wherein the functional variant of the lactate binding protein comprises a lactate optical probe,

preferably, the lactate optical probe comprises lactate binding protein and optically active polypeptide,
more preferably, the optical probe has one or more features selected from the group consisting of:

the lactate binding protein has the sequence shown in SEQ ID NO: 1 or a functional fragment thereof,
the lactate binding protein has mutations at: (1) P189 and/or P190, and optionally (2) M185,
the optically active polypeptide is located at one or more sites of the lactate binding protein selected from the group consisting of: 93/94, 93/95, 93/96, 93/97, 94/95, 94/96, 94/97 , 95/96, 95/97, 96/97, 119/120, 119/121, 120/121, 137/138, 137/139, 137/140, 137/141, 138/139, 138/140, 138 /141, 139/140, 139/141, 140/141, 158/159, 158/160, 158/161, 159/160, 159/161, 160/161, 185/186, 185/187, 185/188 , 185/189, 185/190, 185/191, 186/187, 186/188, 186/189, 186/190, 186/191, 187/188, 187/189, 187/190, 187/191, 188 /189, 188/190, 188/191, 189/190, 189/191, 190/191, 208/209, 208/210, 209/210, 230/231, 230/232 and/or 231/232.

5. A kit for detecting lactate in urine, the kit comprises a reagent for detecting lactate and a urine treatment reagent,
preferably, the reagent for detecting lactate comprises a reagent required for detecting lactate by one or more methods selected from the group consisting of:

chromatography, titration, colorimetry, enzymatic analysis, and optical probe method,
more preferably, the reagent for detecting lactate comprises a reagent for converting, enriching, separating or identifying lactate.

6. The kit according to item 5, wherein the reagent for detecting lactate comprises one or more selected from the group consisting of: antibodies, lactate dehydrogenase, lactate oxidase, lactate binding proteins or functional variants thereof. Phosphate, 3NPH_HCl, EDC, formic acid, acetonitrile, isopropyl alcohol,
the urine treatment reagent comprises: 3NPH_HCl, EDC, buffer; preferably, the buffer is a phosphate-based buffer or Tris.

7. A kit for detecting lactate in urine, comprising a lactate optical probe and a buffer, the optical probe comprising a lactate binding protein and an optically active polypeptide,

preferably, the optically active polypeptide is located in the sequence, N-terminal or C-terminal of the lactate binding protein,
preferably, the buffer is a phosphate-based buffer or Tris, such as HEPES, PBS, etc.

8. The kit according to item 7, wherein the optical probe has one or more features selected from the group consisting of:

the lactate binding protein has the sequence shown in SEQ ID NO: 1 or a functional fragment thereof,
the lactate binding protein has mutations at: (1) P189 and/or P190, and optionally (2) M185,
the optically active polypeptide is located at one or more sites of the lactate binding protein selected from the group consisting of: 93/94, 93/95, 93/96, 93/97, 94/95, 94/96, 94/97 , 95/96, 95/97, 96/97, 119/120, 119/121, 120/121, 137/138, 137/139, 137/140, 137/141, 138/139, 138/140, 138 /141, 139/140, 139/141, 140/141, 158/159, 158/160, 158/161, 159/160, 159/161, 160/161, 185/186, 185/187, 185/188 , 185/189, 185/190, 185/191, 186/187, 186/188, 186/189, 186/190, 186/191, 187/188, 187/189, 187/190, 187/191, 188 /189, 188/190, 188/191, 189/190, 189/191, 190/191, 208/209, 208/210, 209/210, 230/231, 230/232 and/or 231/232.

9. The kit according to any one of items 5 - 8, wherein the kit further comprises: a reagent for detecting mitochondrial m.3243A>G mutation, a reagent for detecting blood glucose of the subject, and/or a reagent for detecting urine glucose of the subject.

10. A method for diagnosing MIDD or identifying MIDD in patients with diabetes comprises: (1) detecting lactate in urine, and (2) comparing same with the control level, wherein lactate level in the urine of MIDD patients is higher than the control level.

The invention will be further described below in specific examples. It should be understood that these examples are illustrative only and are not intended to define the scope of the invention. Unless otherwise stated, the methods and reagents used in the examples are conventional methods and reagents in the art.

Example

Detection method:

1. Protein induced expression and purification

The probe plasmids were transformed into the BL21 (DE3) or JM109 (DE3) expression strain, and the monoclonal clone was picked and cultured in a test tube for primary culture. The next day, the primary cultured bacteria were inoculated into an Erlenmeyer flask at a ratio of 1:100. When $OD_{600}$=0.4-0.6, IPTG with a final concentration of 1 mM was added, and the culture was incubated at 18°C to induce expression of the target protein.

(1) Collect bacterial cells: after 24-48 hours of protein expression, the bacterial cells expressing the protein were harvested by centrifugation at 4000 rpm for 10 minutes in a centrifuge and then resuspended in buffer A.
(2) Ultrasonic fragmentation: the program of the ultrasonic fragmentation instrument is set to ultrasonic for 1 second and then stop for 3 seconds. A working cycle time is 300 seconds. A Φ15 probe is used and the power is 55%.
(3) Centrifugation: When the bacterial cells were sonicated until clear, they were centrifuged at 9600 rpm for 30 minutes at 4 °C in a centrifuge. The supernatant was retained, and the precipitate was discarded.
(4) Column pretreatment: The self-packed nickel ion affinity chromatography column is first rinsed with 5 column volumes (CV) of deionized water, and then equilibrated with 5 column volumes (CV) of buffer A.
(5) Protein loading onto the column: The supernatant obtained after disruption and centrifugation was added to the prepacked nickel column.
(6) Impurity washing: Washing buffer containing 50mM imidazole was added to remove impurity proteins.
(7) Elution: Elution buffer containing 300mM imidazole was added to elute the target protein from the nickel column.
(8) Nickel column treatment: 5 column volumes (CV) of buffer B (a buffer containing 500 mM imidazole) was added to the used nickel column., and then 5 column volumes (CV) of deionized water was added. Finally, the column packing was immersed in 20% ethanol.

Buffer A: 20mM phosphate, 0.5M NaCl, 10mM imidazole.
Buffer B: 20mM phosphate, 0.5M NaCl, 500mM imidazole.
The protein is dissolved in the elution buffer after protein purification through the nickel column, while the buffer needs to be replaced when characterizing or storing the protein. Therefore, desalination treatment needs to be carried out on the purified protein. First, the desalting column needs to be pre - treated. It is rinsed with 5 column volumes (CV) of deionized water and then treated with 5 column volumes (CV) of desalting buffer. Second, the protein to be desalted is added to the desalting column, and the protein is collected. Finally, the desalting column is treated with 10 column volumes (CV) of deionized water.

2. Clinical sample collection

Serum samples from the diabetes and healthy groups were provided by the Sixth People's Hospital Affiliated to Shanghai Jiao Tong University and Shanghai East Hospital. Sample collection and subsequent experiments were approved by the institutional review committee, and informed consent was obtained from all participants.

3. Serum and urine detection methods

For the lactate analysis based on fluorescent protein probe, serum samples were diluted 50- fold or 100-fold in HEPES buffer and urine samples were diluted 20-fold. A 96-well black well plate was used for measurement, and different concentration gradient lactate standards were set. 50 μL of the diluted sample and 50 μL of 0.8 μM probe protein solution

were added. Alternatively, the Echo 650 acoustic liquid handler combined with the BioTek MultifloFX automated dispenser was employed to directly mix 0.5 μL of serum sample or 2.5 μL of urine sample with 100 μL of probe protein (0.4 μM). The fluorescence intensity was measured immediately through the 485BP 20nm or 420BP 10nm excitation and 528BP 20nm emission bandwidth filters of the Synergy neo2 multifunctional microplate reader, and quantitative analysis was performed based on the lactate standard curve.

Data processing of fluorescence detection (taking the FiLa probe as an example)

$$F = F_{Sample} - F_{BLK}$$

$$R(Ratio) = F485 / F420$$

F represents the actual fluorescence intensity of a single channel, $F_{Sample}$ represents the fluorescence intensity of the sample expressing the probe, and $F_{BLK}$ represents the fluorescence intensity of the sample without the probe. $F_{485}$ represents the fluorescence intensity of the fluorescent protein sample excited at 485nm and emitted at 528nm, and $F_{420}$ represents the fluorescence intensity of the fluorescent protein sample excited at 420nm and emitted at 528nm. $Ratio_{FiLa}$ represents the fluorescence intensity ratio of the probe.

The bandwidth BP (band pass) of the filter represents the total range on both sides of the median, 485 BP 20nm is 475-495nm.

$$[Lac] = K_d (R-R_{min}) / (R_{max}-R)$$

[Lac] represents the lactate level; $K_d$ represents the dissociation constant of the FiLa probe; $R_{min}$ and $R_{max}$ represent the fluorescence ratio of the probe protein without adding or adding saturated concentration of lactate respectively; R represents the fluorescence ratio of urine or serum.

4. UHPLC-MS analysis of lactate in serum and urine

Lactate in serum and urine was determined by UHPLC (Agilent, 1290) and triple quadrupole (Agilent, 6460C) mass spectrometer (Analytical Chemistry 93, 5709-5717). 5 μL aliquot of serum or urine sample or standard solution was mixed sequentially with 25 μL of 160 mM 3NPH_HCl solution and 25 μL of 120 mM EDC solution. The mixture was frozen at -20°C for 20 minutes, and then the supernatant was centrifuged for quantitative analysis.

Each 5 μL sample was injected into the Agilent 1290UHPLC system, separated with water, and eluted with a gradient of 0.1% formic acid and acetonitrile/isopropanol (7:3, v/v). Mass analysis for multiple reaction monitoring is performed using ESI negative mode. The paired ions for lactate quantification are 224/137.

Example 1

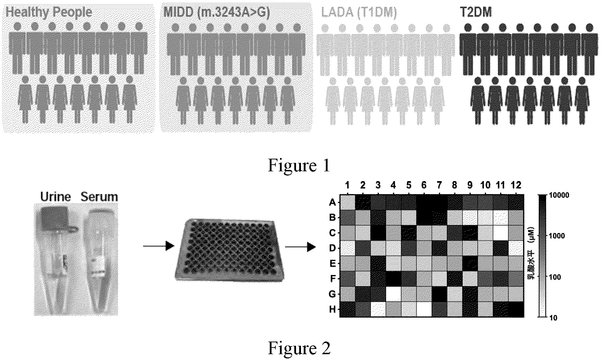

Detect serum samples from patients with latent autoimmune diabetes in adults (LADA, a type of T1DM), T2DM and MIDD (a special type of diabetes caused by mitochondrial m.3243A>G mutation) provided by the partner hospital with 15 patients per disease type. In order to eliminate metabolic differences caused by indicators such as age, gender, height and weight, this example matched patient samples with control samples from healthy people based on age, gender, BMI and other indicators of diabetic patients. The clinical characteristics are as shown in Table 1-4 and Figure 1 below.

Table 1

| Healthy people | | | | | | |
|---|---|---|---|---|---|---|
| Sample Number | Gender | Age | BMI (kg/m$^2$) | HbA1c (%) | Serum Lactate Concentration (μM) | Urine Lactate Concentration (μM) |
| Healthy-1 | female | 39 | 19.7 | 5.8 | 2512 | 90 |
| Healthy-2 | female | 57 | 21.0 | 5.5 | 2301 | 51 |
| Healthy-3 | female | 55 | 21.8 | 6 | 2298 | 40 |
| Healthy-4 | female | 26 | 19.7 | 5.9 | 1822 | 68 |
| Healthy-5 | female | 47 | 24.0 | 5.9 | 1275 | 418 |
| Healthy-6 | female | 33 | 18.7 | 5.4 | 1445 | 139 |

(continued)

| Healthy people | | | | | | |
|---|---|---|---|---|---|---|
| Sample Number | Gender | Age | BMI (kg/m$^2$) | HbA1c (%) | Serum Lactate Concentration ($\mu$M) | Urine Lactate Concentration ($\mu$M) |
| Healthy-7 | female | 59 | 24.5 | 5.6 | 1574 | 53 |
| Healthy-8 | male | 44 | 25.2 | 5.7 | 3997 | 131 |
| Healthy-9 | male | 66 | 19.7 | 6.3 | 2310 | 108 |
| Healthy-10 | male | 56 | 18.5 | 5.4 | 1133 | 122 |
| Healthy-11 | male | 27 | 22.8 | 5.4 | 1682 | 47 |
| Healthy-12 | male | 39 | 20.4 | 5.8 | 2427 | 145 |
| Healthy-13 | male | 45 | 24.8 | 5.6 | 2936 | 117 |
| Healthy-14 | male | 31 | 22.4 | 5.2 | 1151 | 113 |
| Healthy-15 | male | 25 | 19.0 | 5.2 | 2595 | 120 |

Table 2

| Mitochondrial m.3243A>G mutation (MIDD) Patients | | | | | | |
|---|---|---|---|---|---|---|
| Sample Number | Gender | Age | BMI (kg/m$^2$) | HbA1c (%) | Serum Lactate Concentration ($\mu$M) | Urine Lactate Concentration ($\mu$M) |
| MIDD-1 | female | 44 | 16 | 5.4 | 3733 | 164 |
| MIDD-2 | female | 60 | 19.5 | 6.5 | 1822 | 199 |
| MIDD-3 | female | 51 | 21.2 | 6.8 | 2706 | 795 |
| MIDD-4 | female | 28 | 23.6 | 7.8 | 3023 | 500 |
| MIDD-5 | female | 48 | 25.6 | 10.2 | 5127 | 1090 |
| MIDD-6 | female | 30 | 19.8 | 13.9 | 1986 | 303 |
| MIDD-7 | female | 60 | 22.7 | 6.9 | 2644 | 148 |
| MIDD-8 | male | 46 | 23.3 | 10.3 | 4048 | 489 |
| MIDD-9 | male | 68 | 20 | 5.6 | 1792 | 190 |
| MIDD-10 | male | 60 | 18.2 | 6.3 | 2151 | 83 |
| MIDD-11 | male | 26 | 25.4 | 9.8 | 2911 | 293 |
| MIDD-12 | male | 33 | 18.3 | 7.1 | 2359 | 414 |
| MIDD-13 | male | 47 | 17.3 | 6.5 | 2759 | 60 |
| MIDD-14 | male | 31 | 18.5 | 8 | 2457 | 245 |
| MIDD-15 | male | 21 | 19.2 | 8.4 | 2412 | 187 |

Table 3

| Latent Autoimmune Diabetes in Adults (LADA) Patients | | | | | | |
|---|---|---|---|---|---|---|
| Sample Number | Gender | Age | BMI (kg/m$^2$) | HbA1c (%) | Serum Lactate Concentration ($\mu$M) | Urine Lactate Concentration ($\mu$M) |
| LADA-1 | female | 38 | 22.9 | 8.7 | 4702 | 227 |
| LADA-2 | female | 56 | 22.6 | 8.5 | 3697 | 45 |
| LADA-3 | female | 46 | 26.6 | 6.9 | 3027 | 30 |
| LADA-4 | female | 30 | 20.3 | 8.2 | 3281 | 235 |

(continued)

| Latent Autoimmune Diabetes in Adults (LADA) Patients | | | | | | |
|---|---|---|---|---|---|---|
| Sample Number | Gender | Age | BMI (kg/m$^2$) | HbA1c (%) | Serum Lactate Concentration ($\mu$M) | Urine Lactate Concentration ($\mu$M) |
| LADA-5 | female | 47 | 23.3 | 7.3 | 2420 | 127 |
| LADA-6 | female | 31 | 19.0 | 10.1 | 2909 | 211 |
| LADA-7 | female | 53 | 17.5 | 10.2 | 3410 | 98 |
| LADA-8 | male | 39 | 19.0 | 13.9 | 1582 | 36 |
| LADA-9 | male | 58 | 20.5 | 8.4 | 1529 | 155 |
| LADA-10 | male | 61 | 17.6 | 8.5 | 2524 | 10 |
| LADA-11 | male | 23 | 20.4 | 7.1 | 1411 | 170 |
| LADA-12 | male | 35 | 20.3 | 6.3 | 1831 | 120 |
| LADA-13 | male | 40 | 17.3 | 11.9 | 2726 | 40 |
| LADA-14 | male | 29 | 20.4 | 10.7 | 3461 | 47 |
| LADA-15 | male | 29 | 21.2 | 11.3 | 1578 | 352 |

Table 4

| Type II Diabetes Mellitus Patients (T2DM) | | | | | | |
|---|---|---|---|---|---|---|
| Sample Number | Gender | Age | BMI (kg/m$^2$) | HbA1c (%) | Serum Lactate Concentration ($\mu$M) | Urine Lactate Concentration ($\mu$M) |
| T2DM-1 | female | 39 | 21.3 | 6.9 | 2635 | 61 |
| T2DM-2 | female | 55 | 22.5 | 7.4 | 2375 | 58 |
| T2DM-3 | female | 59 | 20.44 | 7.3 | 4621 | 134 |
| T2DM-4 | female | 34 | 26.17 | 10.1 | 1325 | 278 |
| T2DM-5 | female | 42 | 24.3 | 10.3 | 4096 | 99 |
| T2DM-6 | female | 30 | 17.5 | 8.4 | 3139 | 150 |
| T2DM-7 | female | 61 | 21.2 | 13 | 3810 | 75 |
| T2DM-8 | male | 44 | 23.3 | 11.8 | 2766 | 15 |
| T2DM-9 | male | 70 | 20.24 | 6.7 | 4356 | 49 |
| T2DM-10 | male | 58 | 22.8 | 13.8 | 2910 | 178 |
| T2DM-11 | male | 34 | 26.6 | 9 | 3833 | 56 |
| T2DM-12 | male | 26 | 18.3 | 14.7 | 4027 | 100 |
| T2DM-13 | male | 46 | 26.6 | 11.5 | 2114 | 122 |
| T2DM-14 | male | 36 | 19.8 | 7.7 | 3710 | 16 |
| T2DM-15 | male | 21 | 32.8 | 12.7 | 4282 | 269 |

First, a probe-based point-of-care testing (POCT) method for clinical samples was established. The measurement time is less than 1 minute. For a 96 - well plate, only 0.5 $\mu$L of serum or 2.5 $\mu$L of urine is required. The detection flow chart is shown in Figure 2.

Example 2

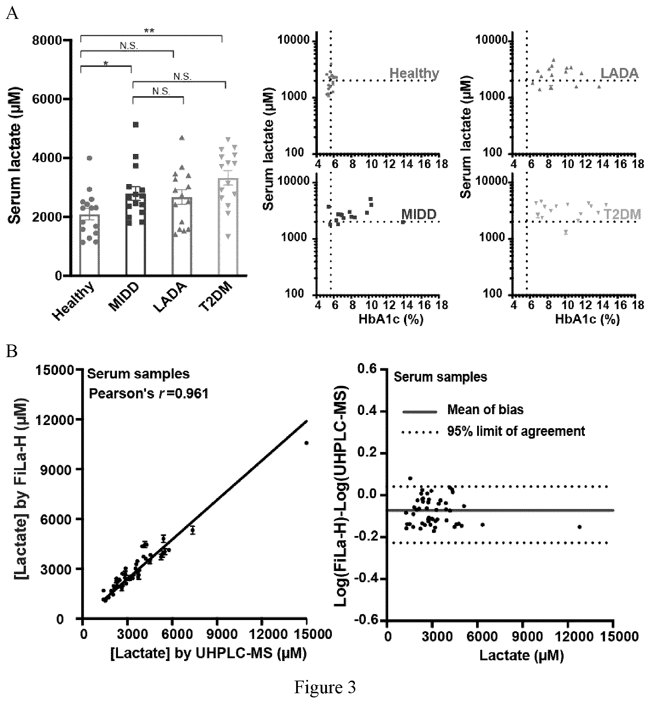

In a random serum detection, the FiLa-H probe (SEQ ID NO: 2) was used for detection. The T2DM group had the highest serum lactate level (3.33±0.25mM), followed by the m.3243A>G mutation group (2.80±0.23mM), LADA group (2.67±0.25mM) and control group (2.10±0.20mM). To validate the accuracy of the probe-based method for lactate detection, this example compared results from ultrahigh-performance liquid chromatography/mass spectrometry (UHPLC-MS) with those from the probe assay. Compared to HPLC-MS, UHPLC-MS has higher resolution, sensitivity and detection efficiency. In this example, correlation analysis and Bland-Altman analysis are used to evaluate the results obtained by the two methods. The results show that the r value of the Pearson correlation coefficient obtained by the correlation analysis of the two methods is as high as 0.961. The Bland-Altmann analysis results show that most of the analysis results fall within the 95% limits of agreement, indicating high consistency and reliability between the two methods, as shown in Figure 3.

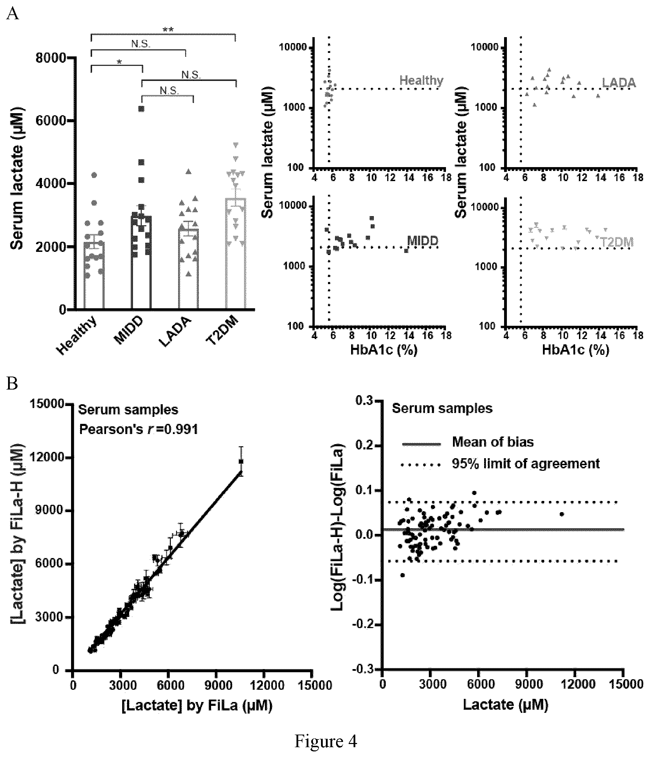

The same results were observed using the FiLa probe (SEQ ID NO: 3) and other probes from the examples in PCT/CN2020/137900, demonstrating strong correlation and consistency, as shown in Figure 4.

Example 3

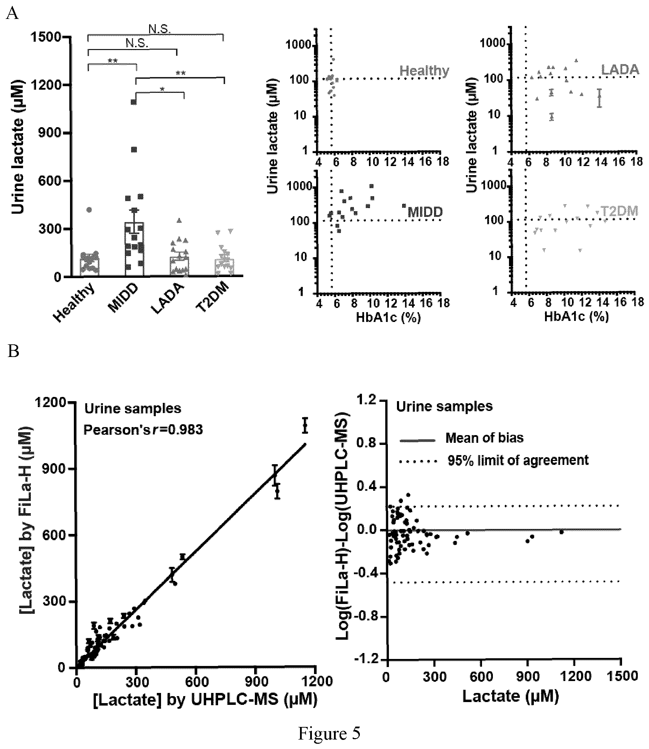

In a random urine detection, the FiLa-H probe was used for detection. The lactate level in the m.3243A>G mutation group was 344±73 μM, which was significantly higher than the other three groups, while there was no significant difference between LADA, T2DM, and the control group (127±25μM vs 111±21μM vs 117±23μM, p>0.5), as shown in Figure 5(A).

The UHPLC-MS method was used for measurement, and correlation analysis and Bland-Altmann analysis were used to evaluate the results measured by the two methods. It showed that the results of the probe detection were consistent with the UHPLC-MS measurement results (Figure 5(B) shown).

Example 4

The FiLa-H probe was used to detect the serum lactate concentration and urine lactate concentration of the subject sample. The results are shown in Table 1-4.

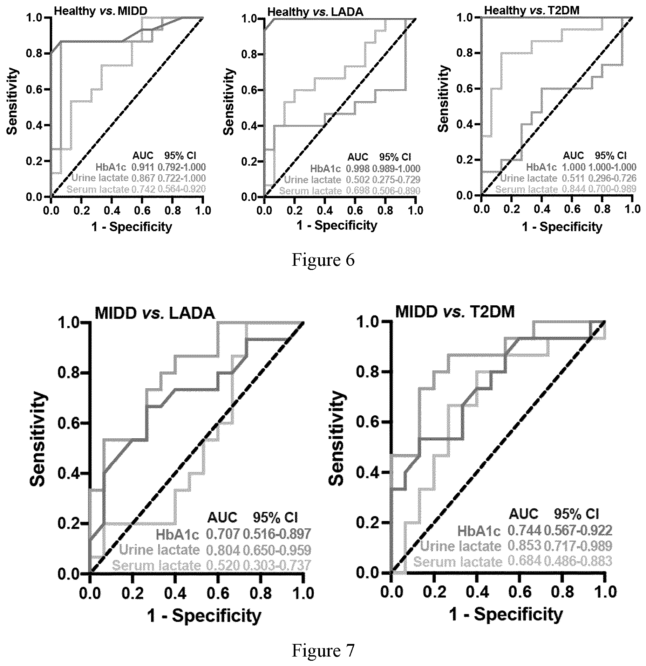

ROC analysis showed that urinary lactate has reasonable discriminating ability between MIDD and healthy controls, with an area under the curve (AUC) of 0.867; while urinary lactate has no significant discriminating ability between LADA and healthy controls (0.502, failure score), and there was also no significant discriminatory ability between T2DM and healthy controls (0.511, failure score) (Figure 6). Therefore, high urinary lactate levels are a significant clinical parameter in patients with MIDD (m.3243A>G), a previously unreported result.

In addition, MIDD patients are often misdiagnosed as T1DM or T2DM due to the lack of significant differentiation in patients' age of onset and clinical manifestations. As shown in Figure 7, the urinary lactate levels of MIDD patients are also significantly different from those of T1DM or T2DM: MIDD and LADA (urinary lactate, 0.804; serum lactate, 0.520) and T2DM (urinary lactate, 0.853; serum lactate, 0.684). Therefore, the urinary lactate characteristic score can help differentiate MIDD from LADA and T2DM.

These results indicate that elevated urinary lactate is closely associated with patients with MIDD and can be used as a potential screening marker for this disease. Re-identify these clinical manifestations of diabetes (abnormally increased urinary lactate and blood glucose) and thus promote rapid screening of MIDD.

**Claims**

1. Use of a reagent for detecting lactate in a urine-derived sample, or a reagent for detecting lactate in a urine-derived sample and a lactate standard, in the preparation of a kit for diagnosing MIDD or for identifying MIDD in diabetic patients.

2. The use according to claim 1, wherein the reagent for detecting lactate comprises a reagent required for detecting lactate by one or more methods selected from the group consisting of: chromatography, titration, colorimetry, enzymatic analysis method, optical probe method.

3. The use according to claim 1 or 2, wherein the reagent for detecting lactate comprises a reagent for converting, enriching, separating or identifying lactate,
preferably, the reagent for detecting lactate comprises one or more reagents selected from the group consisting of: an antibody, a lactate dehydrogenase, a lactate oxidase, a lactate binding protein or a functional variant thereof, formic acid, acetonitrile, isopropyl alcohol.

4. The use according to claim 3, wherein the functional variant of the lactate binding protein comprises a lactate optical probe,

preferably, the lactate optical probe comprises lactate binding protein and optically active polypeptide,
more preferably, the optical probe has one or more features selected from the group consisting of:

the lactate binding protein has the sequence shown in SEQ ID NO: 1 or a functional fragment thereof,
the lactate binding protein has mutations at: (1) P189 and/or P190, and optionally (2) M185,
the optically active polypeptide is located at one or more sites of the lactate binding protein selected from the group consisting of: 93/94, 93/95, 93/96, 93/97, 94/95, 94/96, 94/97 , 95/96, 95/97, 96/97, 119/120, 119/121, 120/121, 137/138, 137/139, 137/140, 137/141, 138/139, 138/140, 138 /141, 139/140, 139/141, 140/141, 158/159, 158/160, 158/161, 159/160, 159/161, 160/161, 185/186, 185/187, 185/188 , 185/189, 185/190, 185/191, 186/187, 186/188, 186/189, 186/190, 186/191, 187/188, 187/189, 187/190, 187/191, 188 /189, 188/190, 188/191, 189/190, 189/191, 190/191, 208/209, 208/210, 209/210, 230/231, 230/232 and/or 231/232.

5. A kit for detecting lactate in urine, the kit comprises a reagent for detecting lactate and a urine treatment reagent,

preferably, the reagent for detecting lactate comprises a reagent required for detecting lactate by one or more methods selected from the group consisting of: chromatography, titration, colorimetry, enzymatic analysis, and optical probe method,
more preferably, the reagent for detecting lactate comprises a reagent for converting, enriching, separating or identifying lactate.

6. The kit according to claim 5, wherein the reagent for detecting lactate comprises one or more selected from the group consisting of: an antibodies, a lactate dehydrogenase, a lactate oxidase, a lactate binding protein or a functional variant thereof, 3NPH_HCl, EDC, formic acid, acetonitrile, isopropyl alcohol,
the urine treatment reagent comprises: 3NPH_HCl, EDC, buffer; preferably, the buffer is a phosphate-based buffer or Tris.

7. A kit for detecting lactate in urine, comprising a lactate optical probe and a buffer, the optical probe comprising a lactate binding protein and an optically active polypeptide,

preferably, the optically active polypeptide is located in the sequence of the lactate binding protein, or at N-terminal or C-terminal of the lactate binding protein,
preferably, the buffer is a phosphate-based buffer or Tris, such as HEPES, PBS, etc.

8. The kit according to claim 7, wherein the optical probe has one or more features selected from the group consisting of:

the lactate binding protein has the sequence shown in SEQ ID NO: 1 or a functional fragment thereof,
the lactate binding protein has mutations at: (1) P189 and/or P190, and optionally (2) M185,
the optically active polypeptide is located at one or more sites of the lactate binding protein selected from the group consisting of: 93/94, 93/95, 93/96, 93/97, 94/95, 94/96, 94/97 , 95/96, 95/97, 96/97, 119/120, 119/121, 120/121, 137/138, 137/139, 137/140, 137/141, 138/139, 138/140, 138 /141, 139/140, 139/141, 140/141, 158/159, 158/160, 158/161, 159/160, 159/161, 160/161, 185/186, 185/187, 185/188 , 185/189, 185/190, 185/191, 186/187, 186/188, 186/189, 186/190, 186/191, 187/188, 187/189, 187/190, 187/191, 188 /189, 188/190, 188/191, 189/190, 189/191, 190/191, 208/209, 208/210, 209/210, 230/231, 230/232 and/or 231/232.

9. The kit according to any one of claims 5 - 8, wherein the kit further comprises: a reagent for detecting mitochondrial m.3243A>G mutation, a reagent for detecting blood glucose of the subject, and/or a reagent for detecting urine glucose of the subject.

10. A method for diagnosing MIDD or identifying MIDD in patients with diabetes comprises: (1) detecting lactate in urine, and (2) comparing the same with the control level, wherein lactate level in the urine of MIDD patients is higher than the control level.

Figure 1

Figure 2

A

B

Figure 3

A

B

Figure 4

A

B

Figure 5

Figure 6

Figure 7

**EP 4 585 923 A1**

<table>
<tr><td colspan="2">INTERNATIONAL SEARCH REPORT</td><td>International application No.<br><br>**PCT/CN2023/116730**</td></tr>
</table>

| A. | CLASSIFICATION OF SUBJECT MATTER |
|---|---|

G01N33/53(2006.01)i

According to International Patent Classification (IPC) or to both national classification and IPC

| B. | FIELDS SEARCHED |
|---|---|

Minimum documentation searched (classification system followed by classification symbols)

IPC:G01N33 G01N30 C12Q1

Documentation searched other than minimum documentation to the extent that such documents are included in the fields searched

Electronic data base consulted during the international search (name of data base and, where practicable, search terms used)

CJFD, CNTXT, CNKI, 万方, WANFANG: 母系遗传性糖尿病, 尿, 乳酸, 糖尿病, 突变, 线粒体, 光学活性多肽, 光学探针, 缓冲液, 磷酸盐, 试剂盒, 碳二亚胺, 硝基苯?肼; ENTXT, VEN, WOTXT, NCBI: lactate, lactic acid, midd, EDC, kit, NPH, urinate, urine.

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 115792205 A (EAST CHINA UNIVERSITY OF SCIENCE AND TECHNOLOGY) 14 March 2023 (2023-03-14)<br>claims 1-10 | 1-10 |
| X | US 2021382071 A1 (HELSINGIN YLIOPISTO) 09 December 2021 (2021-12-09)<br>claims 1-19, and description, paragraphs 9-12, 23, and 44-69 | 1-3, 5, 9, 10 |
| Y | US 2021382071 A1 (HELSINGIN YLIOPISTO) 09 December 2021 (2021-12-09)<br>claims 1-19, and description, paragraphs 9-12, 23, and 44-69 | 3, 4, 6 |
| X | CN 113004420 A (EAST CHINA UNIVERSITY OF SCIENCE AND TECHNOLOGY) 22 June 2021 (2021-06-22)<br>claims 1-10, and description, paragraphs 7-38, 61-66, and 76 | 7-8 |
| Y | CN 113004420 A (EAST CHINA UNIVERSITY OF SCIENCE AND TECHNOLOGY) 22 June 2021 (2021-06-22)<br>claims 1-10, and description, paragraphs 7-38, 61-66, and 76 | 3, 4, 6 |

| ✓ | Further documents are listed in the continuation of Box C. | ✓ | See patent family annex. |
|---|---|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **09 November 2023** | **23 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/116730** |

**C. DOCUMENTS CONSIDERED TO BE RELEVANT**

| Category* | Citation of document, with indication, where appropriate, of the relevant passages | Relevant to claim No. |
|---|---|---|
| X | CN 101825625 A (BEIJING ZHONGSHENG JINYU DIAGNOSTIC TECHNOLOGY CO., LTD.) 08 September 2010 (2010-09-08)<br>description, paragraphs 10-20 | 5 |
| X | CN 101324555 A (SUZHOU ANJ BIOTECH CO., LTD.) 17 December 2008 (2008-12-17)<br>description, pages 2-4 | 5 |
| Y | CN 109917040 A (TSINGHUA UNIVERSITY) 21 June 2019 (2019-06-21)<br>description, paragraphs 6-23 | 6 |
| A | CN 112924608 A (DALIAN INSTITUTE OF CHEMICAL PHYSICS, CHINESE ACADEMY OF SCIENCES) 08 June 2021 (2021-06-08)<br>entire document | 1-10 |
| A | WO 02101356 A2 (MITOKOR et al.) 19 December 2002 (2002-12-19)<br>entire document | 1-10 |
| Y | 丛龙杰 等 (CONG, Longjie et al.). "粪便内中短链脂肪酸柱前衍生化UHPLC-QTOF-MS/MS分析方法研究 (Determination of Medium-and Short-Chain Fatty Acids in Feces by UHPLC-QTOF-MS/MS with Pre-Column Derivatization)"<br>药学学报 (Acta Pharmaceutica Sinica),<br>Vol. 55, No. 1, 31 December 2020 (2020-12-31), pages 131-138<br>abstract, and pages 132-134 | 6 |

Form PCT/ISA/210 (second sheet) (July 2022)

**INTERNATIONAL SEARCH REPORT**

International application No.

**PCT/CN2023/116730**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/CN2023/116730**

| Patent document cited in search report | | | Publication date (day/month/year) | Patent family member(s) | | | Publication date (day/month/year) |
|---|---|---|---|---|---|---|---|
| CN | 115792205 | A | 14 March 2023 | None | | | |
| US | 2021382071 | A1 | 09 December 2021 | EP | 3864417 | A1 | 18 August 2021 |
| | | | | WO | 2020074777 | A1 | 16 April 2020 |
| CN | 113004420 | A | 22 June 2021 | WO | 2021121417 | A1 | 24 June 2021 |
| | | | | US | 2023203506 | A1 | 29 June 2023 |
| | | | | EP | 4079764 | A1 | 26 October 2022 |
| | | | | JP | 2023507210 | A | 21 February 2023 |
| | | | | CN | 113004420 | B | 24 February 2023 |
| CN | 101825625 | A | 08 September 2010 | CN | 101825625 | B | 07 May 2014 |
| CN | 101324555 | A | 17 December 2008 | None | | | |
| CN | 109917040 | A | 21 June 2019 | None | | | |
| CN | 112924608 | A | 08 June 2021 | None | | | |
| WO | 02101356 | A2 | 19 December 2002 | AU | 2002315044 | A1 | 23 December 2002 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211084715
- CN 202011516287
- CN 2020137900 W

**Non-patent literature cited in the description**

- *Biomedical Chromatography*, 2012, vol. 26 (11), 1408-1415
- *Food Chemistry*, 2012, vol. 135 (3), 1078-1082
- *Cell Stem Cell*, 2019, vol. 25 (6), 754-767
- *Annals of Epidemiology*, 2013, vol. 23 (12), 791-796
- *Analyst*, 1972, vol. 97 (151), 142-145
- *Biochemistry Biophysics Reports*, 2016, vol. 5, 35-54
- *Nature Chemical Biology*, 2016, vol. 12 (11), 937-943
- *The Journal of Clinical Investigation*, 2021, vol. 131 (2), e136055
- *Nature*, 2017, vol. 551 (7678), 115-118
- **LACONIC**. *PLos one*, 2013, vol. 8 (2), e57712
- *Scientific Reports*, 2020, vol. 10, 19562
- *Cell Reports Methods*, 2021, vol. 1 (7), 100092
- *Nature Communications*, 2021, vol. 12, 7058
- *Nature Communications*, 2022, vol. 13, 2919
- *Analyst*, 1972, vol. 97 (151), 142-145
- *Analytical Chemistry*, vol. 93, 5709-5717